# EUROPEAN PATENT APPLICATION

(11) **EP 1 170 589 A1**
(43) Date of publication of application: **09.01.2002**
(21) Application number: 00201975.0
(22) Date of filing: 05.06.2000
(51) Int. Cl.: G01N 33/50, C12Q 1/00, G01N 33/569

(54) **Method for histocompatibility testing**

(71) Applicant: Stichting Sanquin Bloedvoorziening, 1066 CX Amsterdam (NL)
(72) Inventor: Hack, Cornelis Erik, 1111 ND Diemen (NL); Goulmy, Elsa Afra Julia Maria, 2343 BC Oegstgeest (NL)
(74) Representative: Prins, Adrianus Willem

(57) **Abstract**

Functional histocompatibility testing wherein stimulator cells, which are lymfocytes of a potential organ transplant donor or recipient that have been treated to become incapable of proliferation, are cocultured with responder cells, which are lymfocytes of a potential organ transplant recipient or donor that are capable of proliferation and include cytotoxic effector cells, and degranulation of said cytotoxic effector cells is determined. The degranulation of cytotoxic effector cells, such as Cytotoxic T Lymfocytes and Natural Killer cells, may be determined by measuring a granule constituent, such as a granzyme or perforin, by means of an immunoassay in the coculture supernatant. This new method of functional histocompatibility testing may be used after a preliminary selection of donor-recipient pairs by means of HLA typing.

## Description

### Field of the Invention

This invention is in the field of transplantation immunology and describes a novel method to assess major and minor histocompatibility mismatches between transplant donors and recipients. Said mismatches may cause major complications following organ transplantation, that is rejection of the graft in vivo by cytotoxic T lymfocytes (CTL) of the recipient, or a graft versus host disease by donor T-lymfocytes in recipients. Application of this invention will facilitate selection of optimal donor-recipient combinations for solid organ, bone marrow or stem cell transplantation.

### Background of the Invention

This invention is directed to transplantation-immunology and to in vitro methods to assess the risk for transplant rejection or graft versus host disease (GVHD) in patients receiving organ transplants. Such patients include those receiving solid organ, bone marrow or stem cell transplants.

The major histocompatibility complex (MHC), also called human leukocyte antigen (HLA) system, consists of a number of membrane proteins that are involved in the presentation of antigenic peptides to various T lymfocyte subsets. Two major classes of MHC molecules are discriminated, class I occurring on all nucleated cells in the body and on platelets, and class II molecules which have a more restricted distribution occurring mainly on so-called antigen presenting cells (APC) such as macrophages, monocytes, B-lymfocytes and dendritic cells. These APC initiate specific immune responses by presenting antigens to T-helper lymfocytes (TH). Both MHC classes share the property to present peptides of antigens to lymfocytes containing matching specific antigen receptors. MHC class I molecules present peptides to antigen receptors on CD8-positive T-cells, that are cytotoxic T lymfocytes (CTL), whereas MHC class II molecules present peptides to CD4-positive lymfocytes, that are TH. The binding of CTL to peptides presented by class I molecules on a cell induces a cytotoxic activity of CTL towards that MHC class I carrying cell leading to lysis of the latter. Binding of TH to peptides presented by HLA class II-positive APC may induce proliferation and differentiation of these TH, in particular when APC interact with the latter cells via other molecules as well (for example B7-molecules on the APC with CD28-molecules on the TH). TH appropriately triggered by APC are able to effectively help other immune cells to generate specific antibodies and CTL and to stimulate macrophages. Thus, MHC molecules play a critical role in the induction of specific immune responses (MHC class II) as well as in the effector function of CTL (MHC class I).

MHC class I molecules consist of a single peptide chain that together with another protein, β2-microglobulin, form a four-domain structure, two domains contributing to a groove-like structure which serves as the binding site for peptides. In contrast, MHC class II molecules consist of two peptide chains, an α- and a β-chain, which similarly to MHC class I molecules, also form a four domain structure. Also in case of class II molecules, two of these domains, one originating from the α-chain and the other from the β-chain, together constitute the peptide binding groove. MHC class I and II molecules are each encoded by at least three different genes, in humans located on chromosome 6 yielding HLA-A, -B and -C, and HLA-DP, -DR and -DQ molecules, respectively.

MHC molecules display a wide allotypic variation within the population, in particular regarding the amino acids that contribute to the peptide binding groove. Hence, most individuals are heterozygous for each MHC molecule and have 6 different MHC class I molecules and at least 6 different MHC class II molecules on their cells. The precise composition of MHC molecules of an individual is called the HLA-fenotype. Notably, the chance that two unrelated individuals share the same HLA-fenotype, is virtually zero. In a transplantation setting MHC-molecules are often called MHC- or HLA-antigens as they frequently give rise to immune reactions in the recipient. Hence, these molecules are further referred to as HLA class I or II antigens.

A number of diseases, in particular renal failure, heart failure, a variety of hematological diseases, various malignancies such as solid tumors, as well as autoimmune diseases are nowadays treated by transplantation of an organ from a non-diseased individual, the donor, into a patient, the recipient. The organs transplanted include kidney, heart, lungs, liver, pancreas, islets of Langerhans or bone marrow. As stated above, the chance that two unrelated individuals have exactly the same HLA antigens, is virtually zero. Hence, the transplanted organs, or allografts, frequently bear one or more HLA antigens not shared by the recipient, i.e. there are HLA mismatches between donor and recipient. These mismatches are not only due to allotypic variation of HLA molecules (mismatches of major HLA antigens), but also by allotypic differences between the endogenous peptides presented by HLA molecules (so-called mismatches of minor histocompatibility antigens [mHags]). Consequently, the different HLA molecules on the cells of the allograft are recognized by T lymfocytes of the recipient and evoke a CTL response to these cells. CTL of the recipient will bind to the differing HLA molecules on the cells of the allograft and lyse these cells leading to rejection and failure of the graft. Transplantation of bone marrow or stem cells from one person to another (allogeneic bone marrow transplantation [BMT]) yields a particular situation in that the lymfocytes, including TH and CTL, of the recipient have been eradicated and replaced by donor lymfocytes. In case of mismatches of major or minor histocompatibility antigens between the bone marrow transplant donor and recipient, donor lymfocytes will induce an immune reaction against the cells of the host, which will lead to the destruction of these cells. This reaction of donor lymfocytes against cells of the host becomes clinically manifest as a graft-versus-host disease (GVHD) and is a severe and sometimes lethal complication of allogeneic BMT. Nowadays, GVHD forms a major obstacle in BMT.

Though significant advances have been made in the treatment of transplant rejection and GVHD, modern immunosuppressive therapy cannot always prevent these complications and often leads to severe immune suppression which sometimes may lead to severe morbidity and sometimes mortality in the recipients as well. Hence, prevention of allograft rejection and GVHD is a major concern in transplantation medicine.

Studies during the last decades have clearly indicated that the incidence of allograft rejection or GVHD is related to the number of HLA mismatches between donor and recipient, i.e. to the number of HLA molecules that are dissimilar between donor and recipient. Hence, in daily practice HLA feno- and genotype of recipient and donor are first determined using the standard serological and molecular histocompatibility typing techniques various. Subsequently, donor-recipient pairs are selected that share as many HLA I and II molecules as is possible.

HLA-typing is not sufficient to select a suitable donor-recipient pair for the following reasons: 1) Unrelated pairs seldomly have the same HLA feno/genotype. Hence, one or even more mismatches between donor and recipient occur frequently in daily practice. 2) Genotypic variants need not necessarily be able to induce an immune response. 3) It has been well established that in addition to MHC class I and II differences, various other antigens, termed minor histocompatibility antigens, may induce transplant rejection or GVHD (Goulmy et al. 1996, N.Engl.J.Med. 334:281). Hence, even a perfect match of MHC I and II between donor and recipient does not guarantee that transplant rejection or GVHD will not develop in the recipient. For these reasons, HLA typing is used to select various donor-recipient pairs, these pairs are then subsequently tested for functional histocompatibility and the best matching pair is finally selected for transplantation (Lie JLWT et al., "Histocompatibility typing procedures for selecting allogeneic hematopoietic stem cell donors"; In: Clinical bone marrow and blood stem cell transplantation, Edited by K. Atkinson, Cambridge University Press, 2nd edition 2000, 1057-1072). The tests for functional histocompatibility are laborious, expensive and their usefulness is still debatable. In view of the still expanding HLA polymorphism combined with the increasing numbers of unrelated donor-recipient combinations, a simple and fast donor selection procedure is needed. The present invention provides such a procedure.

Before explaining the invention in detail, a background of the current assays for functional histocompatibility testing is first given. Histocompatibility between donors and recipients is tested with several functional tests. The oldest method is the so-called mixed lymfocyte reaction or mixed lymfocyte culture (MLC). In case of solid organ transplantation this assay can be performed by culturing irradiated peripheral blood mononuclear cells (PBMC) of the donor, for example 5x10⁴ cells exposed to 20 Gy, with an equal number of non-irradiated PBMC of the recipient for several days at 37°C. The irradiated cells are called the stimulator cells since upon irradiation they cannot proliferate anymore and only serve to stimulate the cells of the recipient, the responding cells, to become activated and to proliferate. After this incubation the proliferative response of recipient cells is measured by an incubation with ³[H]-thymidine for several (for example 12-16) hours. In case of differences in MHC class I or II molecules between the stimulating and the responding lymfocytes in the MLC, the latter will become activated and, amongst others via production of cytokines such as interleukin (IL)-2, proliferate, which will lead to incorporation of ³[H]-thymidine. The incorporation of this radioactive isotope can be assessed by a liquid scintillation counter. The larger the differences in MHC molecules between donor and recipient, the more ³[H]-thymidine is incorporated. In case of BMT a similar kind of MLC is performed except that recipient PBMC are irradiated and the response of donor PBMC to recipient cells is measured.

The MLC assay has been the golden standard to assess histo-incompatibility for many decades. However, this assay has several disadvantages: 1) It measures proliferation in the culture system independently of the nature of the cells. To reduce the possibility that proliferation of irrelevant cells is measured, several laborious controls are included. 2) It is generally assumed that mainly proliferation of TH cells are responding in the MLC. Although activation and proliferation of these cells is essential for the generation of cytotoxic effector responses, measurement of the activation and proliferation of the latter population (i.e. cytotoxic effector cells) may provide more useful information regarding the chance of developing clinical complications following transplantation. 3) In practice MLC is a laborious assay requiring cell culture facilities as well as facilities to work with radioactive isotopes.

To alleviate some of the disadvantages of the MLC, researchers have attempted to modify this assay. Modifications include analysis of the induction of cytokines such as IL-2 or interferon-γ in the MLC, either by assessing mRNA produced by the cells or measurement of protein in cells or supernatant of MLC (Tanaki et al., 1994, Br.J.Haematol. 87:415). These approaches have the advantage that they do not require specific facilities for radiolabeled isotopes, but have the disadvantage that PBMC may produce cytokines upon triggering by a variety of stimuli such as for example low concentrations of endotoxin, which are frequently present in culture media.

Another modification includes the histochemical measurement of the generation of serine esterase activity of the cells during MLC (Maiocchi et al., 1998, Haematologica 83:686). This approach has several disadvantages: 1) The histochemical substrates used in this assay can be converted by many serine-proteases, including several intracellular proteases. 2) The assay requires (laborious) analysis of cells. 3) In particular NK cells and to a lesser extent CTL contain a background level of granzymes, which will be increased in the presence of various cytokines such as IL-2, as may occur in vivo as well as in vitro. Hence, background expression and non-specific induction of synthesis may blur the results of this modified MLC. Presumably for reasons outlined above, the modified MLCs have not replaced the classic MLC measuring ³[H]-thymidine incorporation.

As an alternative for MLC, the determination of the frequency of CTL-precursors (CTL-p) or TH-precursor cells (TH-p) has been advocated to assess histocompatibility between donors and recipients. In these assays dilutions of PBMC are incubated with stimulating cells. The response is measured as ³[H]-thymidine incorporation when TH-p frequency is determined. More often the lysis of stimulating cells is measured with a ⁵¹[Cr]-release assay, which provides a measure of the frequency of CTL-p. The higher the dilution of the responding cells at which significant incorporation of ³[H]-thymidine or ⁵¹[Cr]-release is observed, the higher the frequency of TH-p or CTL-p in the patient (or donor).

The disadvantages of these assays are: 1) They are very laborious to perform and require large numbers of cells. 2) They require special lab facilities in particular since radio-active isotopes are used. 3) The results of these assays show inconsistent relationships with the occurrence of graft rejection or GVHD in recipients.

In conclusion, no perfect test exists to assess the histocompatibility between donor and recipient.

The present invention describes a method for functional histocompatibility testing that obviates most of the concerns discussed above: 1) It directly and specifically measures the effector mechanism, i.e. activation and degranulation of cytotoxic cells, involved in graft rejection or GVHD. 2) It does not require special lab facilities and can be measured with simple Elisa readers. 3) Except that it requires culturing of cells for 96 hours at 37°C, it is easy to perform.

Application of this assay in combination with classical HLA serotyping may offer a new, rapid and cheap method for selection of suitable donor-recipient pairs for transplantation: pairs are selected by classical HLA serology (without expensive HLA genotyping) and functionally tested for histocompatibility by the new assay, i.e. measurement of granzyme-release during MLC.

In case of mismatches of major and minor histocompatibility antigens between donor and recipient, CTL of the recipient recognize the different HLA molecules on the cells of the allograft, become activated and kill these cells. In case of GVHD following allogeneic BMT, CTL, and possibly natural killer (NK) cells, of the donor interact in a similar way with cells in various organs of the recipient. Before explaining the principle of the current invention it is necessary to discuss the molecular mechanisms of the killing machinery of CTL.

Activated cytotoxic T lymphocytes (CTL) can induce apoptosis in their target cells by at least two different mechanisms (Lowin et al., 1994, Nature 370:650; Kägi et al., 1994, Nature 369:31). One pathway leading to apoptosis in target cells involves the Fas-mediated pathway, the other the granule-exocytosis pathway.

The first pathway involves triggering of a membrane protein on the target cell, called Fas (or APO-1), by an appropriate counter-molecule on the membrane of activated CTL, termed Fas-ligand (Fas-L).In the target cell this will lead to the oligomerization of so-called death domains located in the intracellular parts of the Fas-molecules. This oligomerization leads via several other molecules to the activation of effector caspases (cysteine-proteinases with special specificity) in the target cell that subsequently cleave various essential intracellular proteins, which leads to DNA breakdown and apoptotic cell death.

The degranulation pathway comprises a number of proteins, including perforin, granzymes (stands for Granule-associated enzymes) and T cell restricted intracellular antigen (TIA-1), stored in the cytotoxic granules of CTL. Upon contact of CTL with a target cell, these cytoplasmic granules are directed to the contact area after which their constituents are released into the intercellular space. In the presence of extracellular calcium, perforin molecules polymerize into transmembrane channels inserting into the membrane of the target cell (Masson & Tschopp, 1985, J Biol Chem 260:9069; Podack, 1985, Immunol Today 6:21) thereby allowing the other granule proteins, notably granzymes, to enter the target cell. In the cytoplasm of the target cell, granzymes directly, that is without the need for additional signal transduction processes, activate effector caspases, which in turn execute apoptosis. Hence, the release of granzymes by CTL is a key event in most cytotoxic reactions.

We show here that the release of granzymes A or B in the supernatant of MLC correlates with the number of MHC mismatches between donor and recipient and predicts the occurrence of severe GVHD following BMT. Furthermore, the release of granzymes during the co-culturing of donor and recipient lymfocytes can be measured by quantitative and sensitive Enzyme-linked immunoabsorbent assays (Elisa). Hence, the present invention provides a method that is more easy to perform, requires less specialized facilities and is less costly then MLC testing and extensive molecular HLA typing.

The invention allows to identify major and minor histocompatibility antigen differences between the stem cell recipients and their potential donors, and can predict the occurrence of GVHD in patients treated with allogeneic BMT.

### Summary of the Invention

The present invention uses the finding that assessment of the degranulation of CTL in co-cultures of donor and recipient lymfocytes is correlated with the number of MHC mismatches between donor and recipient, and predicts the occurrence of severe GVHD in patients receiving allogeneic BMT. The present invention contemplates a method to measure said degranulation of CTL in these co-cultures.

Herein it is preferred that donor and recipient lymfocytes are co-cultured by mixing donor peripheral blood mononuclear cells (PBMC) with recipient PBMC to yield a so-called mixed lymfocyte culture or MLC. In case reactivity of recipient lymfocytes to donor cells has to be tested, for example for kidney transplantation, donor PBMC are irradiated (stimulating lymfocytes), whereas those of the recipient are not (responding lymfocytes). In case reactivity of donor lymfocytes against recipient cells has to be assessed, as is the case in BMT, recipient PBMC are irradiated. Donor and recipient PBMC are then incubated, for example for 96 hours at 37°C. Thereafter, the supernatant is harvested for measurement of constituents released during degranulation of CTL.

According to the invention, the preferred embodiment to measure degranulation of the CTL during MLR is to quantitate the amount of soluble granzymes, in particular soluble granzyme A or B, in the supernatant. However, also other compounds released by activated CTL, for example perforin, can be measured. Alternatively, the number of granzyme-releasing cells (or other cytotoxic granule component releasing cells) can be assessed, for example by so-called Elispot assays.

A convenient and preferred method to assess the concentration of granzyme A or B in the supernatant of MLC are the Elisas described by Spaeny-Dekking et al. (J.Immunol. 1998;160:3610-6). The more granzyme is released during MLC, the stronger the activation and reactivity of the non-irradiated CTL are.

The invention will be more fully understood after a consideration of the following description of the invention.

### Brief Description of the Drawings

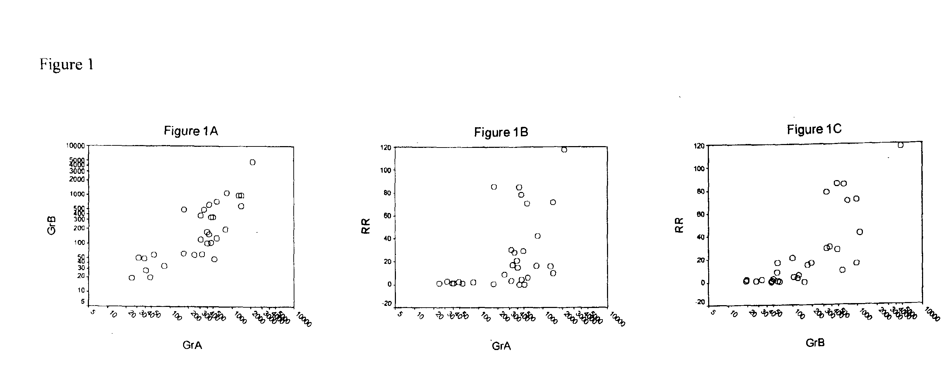

Figure 1: Correlation of granzyme production during MLC with the relative response (RR) of the MLC in 6 sibling and 24 unrelated donor/recipient pairs. Results of the MLC as determined with ³[H]-thymidine incorporation and expressed as percentage (%) RR were related to the release of granzyme A (panel B) or that of granzyme B (panel C). The correlation between the release of granzyme A and B in these MLC is shown in panel A.

In the MLC the stimulating cell population (peripheral blood mononuclear cells; PBMC) was irradiated (20 Gy). In the classic MLC, following incubation for 4 days at 37°C cells were exposed to 2 µCi of ³[H]-thymidine (40-60 Ci/mMol, Amersham, Arlington Heights, IL) for 12 to 16 hours at 37°C whereafter the incorporation of thymidine was measured (as counts per minute, cpm) in a liquid scintillation counter (Beckman, Galway, Ireland). Negative controls consisted of medium or autologous PBMC (responder[recipient]:stimulator [recipient]) alone. In the modified MLC, cells were not exposed to ³[H]-thymidine, but supernatants were collected and tested for the presence of granzyme A and B with Elisas.

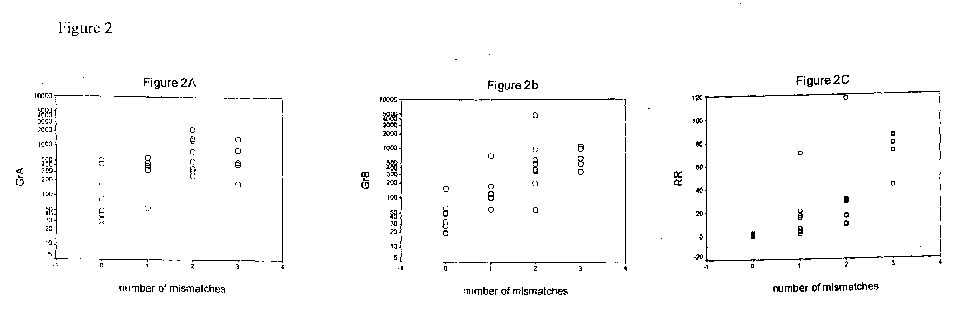

Figure 2: Correlation of granzyme A (panel A) and B (panel B) production levels during MLC with the number of HLA class II mismatches (on the x-axis). Granzyme levels are plotted on the y-axis as the logarithmically transformed geometric mean values. The number of mismatches was derived from HLA typing of donors and recipients with two-colour fluorescence assay using allotypic specific antisera or HLA genotyping using polymerase chain reaction using sequence specific primers. In panel C the correlation between the number of HLA mismatches with the RR in the MLC is shown for comparison.

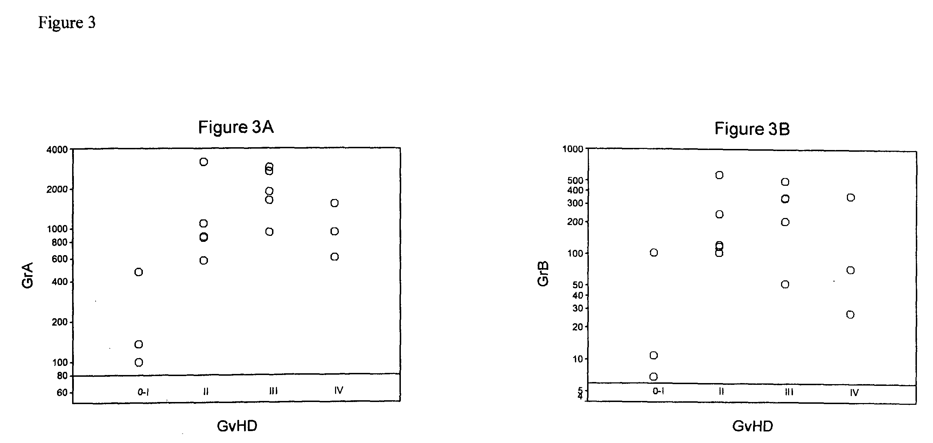

Figure 3: Correlation of granzyme A (panel A) production levels during MLC with the development of GVHD in 16 patients following allogeneic BMT from HLA-identical donors. The correlation between GVHD development and the RR in the MLC is shown in panel B for comparison. The severity of GVHD in the patients was graded according to Glucksberg et al. (Transplantation 1974; 18:295-304).

### Detailed Description of the Invention

This invention provides a new method of functional histocompatibility testing comprising coculturing stimulator cells with responder cells that include cytotoxic effector cells and determining degranulation of said cytotoxic effector cells.

Said stimulator cells either are lymfocytes from a potential organ transplant donor while said responder cells are lymfocytes from a potential organ transplant recipient, or said stimulator cells are lymfocytes from a potential organ transplant recipient while said responder cells are lymfocytes from a potential organ transplant donor.

The organ transplant in question is not particularly limited and may be selected broadly from the group consisting of solid organ transplants, bone marrow transplants and stem cell transplants. Solid organ transplants comprise transplants of kidney, liver, heart, lung, pancreas, islets of Langerhans, etcetera.

The cells used as stimulator cells lack the capability of proliferation. Any treatment destroying the capability of proliferation of the cells may be applied. Practically, such treatment comprises irradiation with an appropriate amount of gamma radiation.

The term "cytotoxic effector cells" used herein refers in particular to cytotoxic T lymfocytes (CTL) and/or natural killer cells (NK). The term distinguishes from other types of lymfocytes, such as in particular T helper lymfocytes.

According to this invention, degranulation of cytotoxic effector cells is determined preferably by measuring in the coculture supernatant a granule constituent. The granule constituent preferably is a granzyme, such as granzyme A or granzyme B, or both. However, any granule constituent may be measured instead, such as for example perforin. Practically, the granule constituent is measured by an immunoassay, such as by an Enzyme-Linked ImmunoSorbent Assay (ELISA).

The invention also provides a method of assessing histo-(in)compatibility between potential organ transplant donors and recipients, comprising HLA typing followed by functional histocompatibility testing for selected donor-recipient pairs, wherein said functional histocompatibility testing is carried out by the method described herein. Although the selection of donor-recipient pairs may be done on the basis of any HLA typing method, including HLA genotyping, it is on practical grounds preferred to make the selection on the basis of HLA serotyping.

The present invention is based on the realization that constituents from the granules of CTL or NK cells are released during MLC in the supernatant and that such release closely reflects the cytotoxic activity of the responder (donor or recipient) against the cells of the stimulator (recipient or donor). Measurement of these constituents in the supernatant of MLC is related to the number of HLA mismatches and to the occurrence and severity of GVHD in recipients of bone marrow allografts transplants. This is illustrated by assessing the release of granzymes, but by no means should this invention be constructed so narrowly that only granzymes are intended to fall within the scope of this invention. Virtually every constituent released during MLC from the cytotoxic granules is intended to fall within the scope of this invention.

The most widely accepted method for testing histocompatibility between donors and recipient is the MLC, which is discussed in the previous paragraphs. In the MLC, cells from donor and recipient are co-cultured for 96 hours at 37°C, whereafter proliferation of the responding cells is measured by assessing incorporation of ³[H]-thymidine. The responding cells can be any cell that is proliferating in this system. The present invention also comprises co-culture of stimulating and responding lymfocytes, but measures parameters that are not only more easy to assess (with ELISA) but also more directly reflect activation of the cytotoxic T cells, that are the cells that in vivo cause rejection or GVHD. Thus in comparison with the current methods, the invention provides a method that is more easy to perform and more specifically reflects the basic pathogenic mechanism underlying transplant rejection or GVHD.

The invention will now be illustrated with some examples. These examples are focussed on the application of the invention to assess histo-incompatibility between donors and acceptors of allogeneic BMT.

### Examples

### Material and methods

Preparation of peripheral blood mononuclear cells (PBMC): Blood samples were obtained after informed consent from donors and patients after treatment for their disease but before any conditioning for BMT. PBMC were separated from heparinized venous blood by density gradient centrifugation (Nycomed Pharma AS, Oslo, Norway). Cells were washed twice and resuspended in culture medium which consists of RPMI 1640 medium (Gibco BRL, Life Technologies Ltd, Paisley, Scotland) supplemented with 2 mM/l L-glutamine (Gibco), 1% antibiotics (Gibco) and 20% heat-inactivated human serum.

MLC: Donor PBMC (5x10⁴) were co-cultured with 5x10⁴ irradiated (20 Gy) patient PBMC in 200 *µ*l culture medium at 37°C in a humidified 5% CO₂/95% air atmosphere. On day 4, supernatants for GrA and GrB measurements were harvested from each well and stored at -20°C until use. Thereafter, cells were exposed to 2 *µ*Ci of ³[H]-thymidine (40-60 Ci/mMol, Amersham, Arlington Heights, IL) for 12-16 hours, harvested and ³[H]-thymidine uptake was measured in a liquid scintillation counter (Beckmann, Galway, Ireland). Results are expressed as median counts per minute of triplicate values. The % RR of the MLC was calculated as follows:
The % RR in the classic MLC was calculated according to the formula: cpm responder[recipient] : cpm stimulator[donor] - cpm responder[recipient] / cpm responder[recipient] : cpm stimulator[pool] - cpm responder[recipient] : cpm stimulator[recipient] x 100%. The pool represents the response of recipient cells to three different HLA-mismatched donors, for the calculation of % RR the median of these three analyses was used. The reference value is the median response to stimulation by unrelated controls.

HLA-typing: All patients and donors were typed by serology for HLA class I and class II. HLA-A, -B, -C typings were done using the standard NIH lymphocytotoxicity assay and HLA-DR and -DQ typing was performed with the two colour fluorescence assay using allo-antisera. HLA genotypes were determined by polymerase chain reaction (PCR) amplification with sequence-specific primers for HLA-DRB1, 3, 4, 5 and -DQB1. HLA-DPB1 typing was done with PCR-sequence-specific oligonucleotides.

ELISA for GrA and GrB: Purification of the monoclonal antibodies (mAb) and the ELISA for GrA and GrB were performed as described (Spaeny-Dekking et al. 1998, J.Immunol. 160: 3610). Briefly, microtiter plates were coated with monoclonal antibody (mAb) against granzyme A or B, preferably mAb GA29 or mAb GB11 (Dept. of Immune Reagents of the Central Laboratory of the Netherlands Red Cross Blood Transfusion Service (CLB), Amsterdam, the Netherlands), respectively, at a concentration of 0.5 *µ* g/ml mAb GA 29 (for GrA) or GB 11 (for GrB) for 16 h at 4°C. Residual binding sites were blocked by a 45-min incubation with PBS/2% (v/v) cow milk. Small aliquots of MLC supernatants are then mixed with high performance Elisa buffer (CLB) and incubated in the mAb-coated plates for 1 h. Finally, bound granzyme A or B are detected by an incubation with biotinylated mAb against the appropriate granzyme, preferably mAb GA28 or mAb GB10 (CLB) in 1% (v/v) normal mouse serum, followed by an incubation with polymerized horse radish peroxidase coupled to strept-avidin (CLB). The reaction is visualized by incubation with a peroxidase substrate, preferably 3,3',5,5'-tetramethylbenzidine (100 *µ*g/ml; Merck, Darmstad, Germany) and 0.003% (v/v) H₂O₂ in 0.11 M sodium acetate buffer (pH 5.5). After stopping the reaction by adding an equal volume of 2 M H₂SO₄, absorbance at 450 nm was determined by a Titer-Tek Multiscan plate reader (Labsystems, Helsinki, Finland).

Statistical analysis: Descriptive statistics are given as median and 25%-75% percentile range. The Spearman correlation coefficient is used to assess the mutual correlation of GrA, GrB, RR and the total number of HLA class II mismatches. The test of Mann-Whitney is used to compare the levels of GrA, GrB and RR between subgroups defined by mismatch or GVHD.

To avoid problems in the statistical analysis caused by the intra-pair correlation of the measurement the "average" value of GrA, GrB and RR in each pair was computed and used for further analysis. In the light of the skewness of the data the geometric mean was used as an "average" for GrA and GrB.

To handle the zero-values of RR, we first added 1% to all RR measurements, next took the geometric mean and finally subtracted 1% again to bring it back to the original scale.

### Results

In one cohort of 6 sibling and 24 unrelated patient/ donor pairs, GrA and GrB production levels were measured in the supernatants of pre-transplant MLC and correlated with the RR of MLC, and with the number of HLA class II mismatches. From this cohort of patient/donor pairs, only one combination was actually selected for BMT. In another cohort of 16 HLA-identical sibling patient/donor pairs, GrA and GrB production levels were measured in the supernatants of pre-transplant MLC and correlated with the development of acute GVHD.

*Correlation of GrA and GrB production levels with RR of MLC*. MLC from each patient/donor pair were established in the graft-versus-host and the host-versus-graft direction. In both latter directions, the RR of MLC and the GrA and GrB production levels in the supernatants of MLC were determined. Figure 1 shows the correlation of the mean GrA and GrB production levels of the 30 patient/donor pairs with the RR of the MLC. GrA and GrB production levels significantly correlate with each other (r=0.78, p=<0.001, Figure 1C) and with the RR of the MLC (r=0.53, p=0.003, Figure 1A, and r=0.79, p=<0.001, Figure 1B).

*Correlation of GrA and GrB production levels with the number of HLA class II mismatches*. From the 30 patient/donor pairs studied, 6 sibling and 3 unrelated patient/donor pairs did not display any mismatch in the HLA class II antigens. Of the 21 patient/donor pairs displaying HLA class II mismatches, 8 patient/donor pairs showed one HLA class II mismatch (either HLA-DRB1, -DQB1 or -DPB1), 8 patient/donor pairs two HLA class II mismatches and 5 patient/donor pairs three HLA class II mismatches. Figure 2 shows the correlations of the GrA and GrB production levels with the number of HLA class II mismatches (r=0.60, p=0.00, r=0.81, p=<0.001 respectively) and the correlation of the RR of MLC with the number of HLA class II mismatches r=0.87, p=<0.001. In detail, the median GrA production levels are 49 pg/ml (25-75% percentiles: 40-169 pg/ml) in supernatants of MLC of the patient/donor pairs without HLA class II mismatches, increase to 397 pg/ml (25-75% percentiles: 320-460 pg/ml; p=0.27 against no HLA class II mismatch) in the patient/donor pairs with one HLA class II mismatch and further increase to 469 pg/ml (25-75% percentiles: 318-1306 pg/ml; p=0.005 against no HLA class II mismatch) in the patient/donor pairs with two HLA class II mismatches. The GrA levels of the patient/donor pairs with three HLA class II mismatches (median 466 pg/ml GrA, 25%-75% percentiles: 435-826; p=0.014 against no HLA class II mismatch) were not higher than the GrA levels of the patient/donor pairs with two HLA class II mismatches (figure 2A).

Similar results were obtained for the GrB production levels. The GrB production levels of the patient/donor pairs without HLA class II mismatches were low (median 46 pg/ml GrB, 25-75% percentiles: 27-51 pg/ml); higher in the patient/ donor pairs with one HLA class II mismatch (median 103 pg/ml GrB, 25-75% percentiles: 59-125 pg/ml; p=0.007 against no HLA class II mismatch) and in the patient/donor pairs with two mismatches (median 374 pg/ml GrB, 25-75% percentiles: 195-587 pg/ml; p=0.001 against no HLA class II mismatch). In the patient/donor pairs with three HLA class II mismatches a further rise of the median GrB levels to 623 pg/ml (25-75% percentiles: 483-977 pg/ml; p=0.003 against no HLA class II mismatch) was observed (figure 2B).

The median RR of the MLC in the patient/donor pairs without HLA class II mismatches was low (1%, 25-75% percentiles: 0.4-1.8%), significantly increased to 6.1% (25-75% percentiles: 3.0-17%; p=0.002 against no HLA class II mismatch) in the patient/donor pairs with one HLA class II mismatch, to 16% (25-75% percentiles: 10-29%; p=0.001 against no HLA class II mismatch) in the patient/donor pairs with two HLA class II mismatches and further increased to 78% (25-75% percentiles: 72-85%; p=0.003 against no HLA class II mismatch) in the patient/donor pairs with three HLA class II mismatches (figure 2C).

*Correlation of GrA and GrB production levels with the development of acute GVHD.* In another cohort of 16 HLA identical patient/donor pairs, GrA and GrB production levels were measured in the supernatants of MLC established in the graft-versus-host direction and correlated with the development of acute GVHD. Figures 3A shows the GrA production levels of 3 patients without acute GVHD (grades 0-I), 5 patients with acute GVHD grade II, 5 patients with acute GVHD grade III and 3 patients with acute GVHD grade IV.

The median GrA levels in the supernatants of MLC of patients without acute GVHD (grades 0-I) were low (median 137 pg/ml GrA, 25-75% percentiles: 100-475 pg/ml) and significantly increased in patients with acute GVHD grades II-IV (median 1103 pg/ml GrA, 25-75% percentiles: 880-1893 pg/ml; p=0.004; Figure 3A).

Similarly, the median GrB production levels in the supernatant of MLC of 3 patients without acute GVHD (grades 0-I) were low (11 pg/ml GrB, 25-75% percentiles: 7-104 pg/ml) and significantly increased in the 13 patients who developed acute GVHD grades II-IV (207 pg/ml GrB; 25-75% percentiles: 105-348 pg/ml; p=0.025; results not shown).

## Claims

1. A method of functional histocompatibility testing comprising coculturing stimulator cells with responder cells that include cytotoxic effector cells and determining degranulation of said cytotoxic effector cells.

2. A method according to claim 1 wherein said stimulator cells are lymfocytes from a potential organ transplant donor and said responder cells are lymfocytes from a potential organ transplant recipient, or said stimulator cells are lymfocytes from a potential organ transplant recipient and said responder cells are lymfocytes from a potential organ transplant donor.

3. A method according to claim 1 wherein said stimulator cells are peripheral blood mononuclear cells (PBMC) from a potential organ transplant donor and said responder cells are effector immune cells from a potential organ transplant recipient, or said stimulator cells are peripheral blood mononuclear cells from a potential organ transplant recipient and said responder cells are effector immune cells from a potential organ transplant donor.

4. A method according to claim 2 or claim 3, wherein said organ transplant is selected from the group consisting of solid organ transplants, bone marrow transplants and stem cell transplants.

5. A method according to any one of claims 1-4, wherein said stimulator cells lack the capability of proliferation.

6. A method according to claim 5, wherein said stimulator cells have been irradiated to destroy their capability of proliferation.

7. A method according to any one of claims 1-6, wherein said responder cells are cytotoxic T lymfocytes (CTL) and/or natural killer cells (NK).

8. A method according to any one of claims 1-7, wherein degranulation of cytotoxic effector cells is determined by measuring a granule constituent in the coculture supernatant.

9. A method according to claim 8, wherein said granule constituent is a granzyme.

10. A method according to claim 9, wherein said granzyme is granzyme A and/or granzyme B.

11. A method according to claim 8, wherein said granule constituent is perforin.

12. A method according to any one of claims 8-11, wherein said granule constituent is measured by an immunoassay.

13. A method according to claim 12, wherein said immunoassay is an Enzyme-Linked ImmunoSorbent Assay (ELISA).

14. A method of assessing histo-(in)compatibility between potential organ transplant donors and recipients, comprising HLA typing followed by functional histocompatibility testing for selected donor-recipient pairs, wherein said functional histocompatibility testing is carried out by the method of any one of claims 1-13.

15. A method according to claim 14, wherein said donor-recipient pairs are selected on the basis of HLA serotyping.
